Europäisches Patentamt

European Patent Office

Office européen des brevets

(11) Publication number: **0 269 278 B1**

# EUROPEAN PATENT SPECIFICATION

(45) Date of publication of patent specification: **15.04.92**  (51) Int. Cl.⁵: **C07D 215/56**, **A61K 31/47**

(21) Application number: **87309600.2**

(22) Date of filing: **29.10.87**

The file contains technical information submitted after the application was filed and not included in this specification

(54) **A water-soluble adduct of norfloxacin.**

(30) Priority: **30.10.86 IL 80459**

(43) Date of publication of application:
**01.06.88 Bulletin  88/22**

(45) Publication of the grant of the patent:
**15.04.92 Bulletin  92/16**

(84) Designated Contracting States:
**AT BE CH DE ES FR GB IT LI NL SE**

(56) References cited:
**EP-A- 138 018**
**EP-A- 0 067 666**
**EP-A- 0 084 011**
**DE-A- 944 518**
**US-A- 3 761 592**

(73) Proprietor: **ABIC LTD.**
**21 Hayozma Street**
**Ramat-Gan(IL)**

(72) Inventor: **Simonovitch, Haim**
**21 Schmaryahu Lewin Street**
**Rishon Le Zion(IL)**

(74) Representative: **Sanderson, Laurence Andrew et al**
**SANDERSON & CO. European Patent Attorneys 34, East Stockwell Street**
**Colchester Essex CO1 1ST(GB)**

## Description

This invention relates to a water soluble adduct of 1-ethyl-6-fluoro-1,4-dihydro-4-oxo-7-(piperazinyl)-3-quinolinecarboxylic acid (hereinafter called "Norfloxacin").

Adduct in connection with the present invention means an adduct proper, a salt (including addition salt), complex, etc.

Norfloxacin, which is described and claimed in U.S. Patent Specifications Nos. 4,146,719 and 4,292,314, is a very effective antibacterial agent having a broad spectrum of activity. However, it has a very low water solubility at the normally used pH range, i.e. 6 to 10.

In many cases it is advantageous to apply a medicament by way of injections, solutions, infusions, etc. Moreover, it is well known that when livestock or fowl are ill the medicament is advantageously administered in the drinking water. As the result of its low water solubility, the use of Norfloxacin in the above indicated manner is thus rather restricted or even impossible.

Certain Norfloxacin salts are known, inter alia from the following publications:

U.S. Patent Specification No. 4,530,928 describes and claims a complex of Norfloxacin with guanidinum carbonate. However, the solubility of said complex in water is also rather low.

Israeli Patent Specification No. 66,026 describes and claims the galacturonic, aspartic, glutamic and gluconic salts of Norfloxacin. The solubility of said salts in water is much better. However, the preparation of said salts is by way of lyophilisation and is expensive.

Said Israeli Patent Specification also describes other salts of Norfloxacin. However, it is explicitly stated that said other salts cannot be used for parenteral purposes, i.e. some of them are not sufficiently water-soluble in the required pH range, some are difficult to manufacture by lyophilisation (acetate) or the acid part cannot be consistently obtained in a satisfactory grade (lactate).

From West German Specification No. 3,333,719 there is known a water-soluble composition comprising the lactate of Norfloxacin together with a further acid which does not cause any precipitation. This composition also is not satisfactory, for besides the reason given above it requires the use of a further acid which complicates its preparation.

For completeness, it possibly deserves mention that the anti coccidial and growth-promoting effects of certain cycloaliphatyloxy- and -mercapto-4-hydroxy-3-quinoline-carboxylic acids have been disclosed in United States Patent Specification No. 3,761,592; while processes for the production of solutions of 1,3-substituted xanthines using derivatives of pyridine carboxlyic acids as solubilizer have been disclosed in West German Patent Specification No. 944,518.

Against that background it has been desirable to find a water-soluble Norfloxacin derivative which overcomes the above drawbacks. Such a derivative should, inter alia, be suitable in the veterinary field, e.g. in the treatment of fowl. It should be relatively inexpensive and easy to manufacture, be biostable on being dissolved in water and have a high solubility so that a commercially feasible water concentrate can be prepared which can,if desired, be further diluted. Moreover, said derivative should, after having been administered internally, ensure an adequate blood level.

A surprisingly good derivative of Norfloxacin has now been discovered.

Accordingly, the invention provides nicotinic acid:Norfloxacin 1:1 adduct.

The invention also provides a process for preparing the adduct, which process comprises reacting Norfloxacin or a different adduct thereof with nicotinic acid or a salt thereof (preferably by reacting Norfloxacin with nicotinic acid) to form the desired adduct.

The reaction is usually conducted in the desired ratio in a suitable solvent, which advantageously is boiling ethanol.

The invention also provides a pharmaceutical composition containing the adduct as active compound. The composition may, if desired, also contain any other suitable additional active compound and/or carrier. The carrier can advantageously be water.

It is a specific feature of the invention to provide an aqueous solution containing the adduct, for instance at least 5%, for example at least 10%, particularly at least 15%, and especially at least 20% by weight of the adduct. The aqueous solution generally contains 0.01 to 25% by weight of the adduct.

The invention also provides the adduct or pharmaceutical composition for use in a method of treatment of the human or animal body by therapy. The adduct, particularly dissolved in water, is particularly useful in this way against bacterial infections.

Adducts of nicotinic acid with related acids, e.g. that of Enoxacin and of Pefloxacin, have been prepared. However, these other adducts have a very low solubility in water. It is therefore surprising that the nicotinic acid-Norfloxacin adduct overcomes the drawbacks mentioned above.

The present adduct retains the broad spectrum of activity of Norfloxacin, is very well water-soluble and, after oral administration, achieves adequate blood levels. Moreover, its toxicity is low. The adduct has the additional advantage that it uses nicotinic acid which is relatively cheap and is known in itself as being a naturally-occurring vitamin.

The present adduct can be administered in a wide variety of therapeutic dosage forms, e.g. by oral administration in the form of tablets or capsules, dissolved in drinking water, as syrup, by intramuscular or intravenous injection or infusion, as ophthalmic solution or ointment, as spray for topical and ear infections, etc. The dosage forms can be conventional in themselves and can be prepared in conventional ways.

The present adduct can be prepared in ways conventional in themselves. It is advantageously prepared by reacting Norfloxacin or a different adduct thereof with nicotinic acid or a salt thereof in a suitable solvent, preferably in boiling ethanol.

The invention is illustrated by the following Examples. While the invention is illustrated for convenience with reference to fowl, it will be understood that its use is not restricted thereto.

Example 1

10 g of Norfloxacin were added to 20 ml of ethanol. The mixture obtained was then stirred and heated to reflux. 4.2 g of nicotinic acid, previously dissolved in 5 ml of hot ethanol, were added in one portion to said mixture and the resulting suspension was heated to reflux, upon which all solids dissolved. After a short time at reflux, the adduct began to separate. The mixture was stirred, cooled to room temperature and finally in an ice bath to yield the nicotinic acid-Norfloxacin 1:1 adduct in 88% yield; m.p. 232°-233°C.

| The analysis was calculated for $C_{22}H_{23}FN_4O_5$: | | | |
| --- | --- | --- | --- |
| Calculated: | C: 59.72%; | H: 5.20%; | N: 12.66% |
| Found: | C: 59.42%; | H: 5.26%; | N: 12.70%. |

I.R. (KBr cm$^{-1}$): 3075, 3000, 2980, 2960, 1715, 1680, 1480, 1350, 1280, 1030, 980, 910, 830, 810, 755, 710, 700, 635.

N.M.R. ($D_2O$): $\delta$ = 3.60 (8H.S); 1.5 (3H, t); 4.3 (2H g); 6.84 (1H, d. JFH 7 $H_z$); 7.22 (1H, d, JFH 13.5 $H_z$); 7.46 (1H, ddd); 8.22 (1H, ddd); 8.44 (1H, S); 8.54 (1H, dd); 8.85 (1H, dd).

M.S. (CI): m/e 320 (protonated Norfloxacin); m/e 124 (protonated nicotinic acid).

$LD_{50}$ (mice oral) > 10 g/kg

$LD_{50}$ (chicken oral) > 10 g/kg

The solubility of the adduct at 25°C in water is 250 mg (equivalent to 180.4 mg Norfloxacin base)/ml.

A solution of 15% in water gives a pH of 5.8.

A solution of 4% in water gives a pH of 6.0.

Example 2

The following water-soluble powder was prepared by well blending:

30 g     of the adduct of Example 1

6 g      sucrose

6 g      ascorbic acid.

Example 3

The following water-soluble powder was prepared by well blending:

30 g     of the adduct of Example 1

12 g     ascorbic acid

0.39 g   nicotinic acid.

Example 4

Comparative Minimal Inhibitory Concentrations (MIC) of several antibacterial quinolones for a wide range of avian pathogenic bacteria.

a. Stock cultures of the bacteria were maintained on nutrient agar and were sub-cultured periodically to ascertain their viability.

b. Stock solutions, 5 mg/ml, were prepared from each drug. The water soluble drugs were dissolved in sterile distilled water and the water insoluble drugs were dissolved in warm (60°C) DMSO. The solutions were prepared on the day on which the assay was conducted.

c. The MIC of each drug was determined by the procedure described in Washington, Jr. J.A. & Stutter, V.L. (1980) "Dilution susceptibility test: agar and macro-broth dilution procedures" in Manual of Clinical Microbiology, Lennette, E.H. Balows, A. Hauser, W.J. & Truant, J.P., editors, 3rd edition, pp 450-453, American Society for Microbiology, Wash. D.C., employing the serial two-fold dilution method and Mueller-Hinton agar (Difco) at pH 7.2. Concentrations tested ranged between 50 $\mu$g/ml and 0.01 $\mu$g/ml. Overnight nutrient broth (Difco) cultures from each bacterial isolate were diluted in 1:100 in sterile saline and approximately 0.01 ml of the diluted culture was placed on the surface of the agar using a modified Steers inoculation device. The bacterial inoculum comprised $5 \times 10^4$ to $1 \times 10^5$ organisms. Inoculated agar plates were incubated aerobically at 37°C for 18 hours. The MIC was recorded from the lowest drug concentration in the agar which, after incubation, upon visual inspection completely inhibited growth.

The MIC values are tabulated in Table I the columns of which indicate:

A: Organisms and ATCC code No.

B: MIC of the compound of Example 1

C: MIC of cinoxacin

D: MIC of pipemidic acid

E: MIC of flumequin

F: MIC of norfloxacin

G: MIC of oxolinic acid.

Table I

| No. | A | B | C | D | E | F | G |
|---|---|---|---|---|---|---|---|
| 1 | Enterobacter Cloaca (23355) | 0.02 | 3 | 0.4 | 0.2 | <0.01 | 0.1 |
| 2 | E.coli (25922) | 0.02 | 3 | 0.4 | 0.2 | <0.01 | 0.1 |
| 3 | Kleb. Pneumonia (13883) | 0.05 | 3 | 1.5 | 0.4 | 0.02 | 0.2 |
| 4 | Prot. Vulgaris (13315) | 0.1 | 3 | 1.5 | 0.2 | 0.01 | 0.1 |
| 5 | Ps. aeruginosa (27853) | 1.5 | >50 | 50 | 50 | 0.8 | 12 |
| 6 | Sal. typhimurium (14028) | 0.05 | 6 | 6 | 0.2 | 0.1 | 0.2 |
| 7 | Serratia marcescens (8100) | 0.05 | 6 | 1.5 | 0.2 | 0.02 | 0.2 |
| 8 | Staph. aureus (25923) | 1.5 | >50 | 50 | 1.5 | 1.5 | 1.5 |
| 9 | Staph. epidermidis (12778) | 0.4 | >50 | 6 | 1.5 | 0.2 | 3 |
| 10 | Strep. pyogenes (19165) | 0.1 | 3 | 3 | 12 | 0.2 | 12 |
| 11 | Strep. faecalis (33186) | 3 | 50 | 50 | 3 | 3 | 12 |
| 12 | Acinobacter colcoaceticus (19606) | 6 | 50 | >50 | 0.8 | 6 | 0.8 |
| 13 | Citrobacter-freund (8090) | 5 | 50 | >50 | 0.2 | 6 | 0.8 |
| 14 | Enterobacter aerogenes (13048) | 0.05 | 6 | 6 | 0.8 | 0.05 | 0.4 |
| 15 | Shigella flexner (12022) | 6 | 50 | >50 | 0.2 | 6 | 0.8 |
| 16 | Shigella sonnei (25931) | 3 | 25 | 50 | 0.2 | 3 | 0.4 |
| 17 | Strep. faecalis (19433) | 3 | >50 | >50 | 25 | 3 | 25 |
| 18 | Strep. agalactiae | 3 | >50 | >50 | 25 | 3 | 50 |
| 19 | Past. multocida | 6 | 25 | >50 | 0.2 | 6 | 0.4 |
| 20 | Staph. aureus (25178) | 0.8 | >50 | 12 | 1.5 | 0.4 | 1.5 |
| 21 | Staph. aureus (8096) | 0.8 | >50 | 12 | 1.5 | 0.8 | 15 |
| 22 | Past multocida ) | >0.01 | 6 | 1.5 | 0.2 | 0.02 | 0.4 |
| 23 | "      " ) Various | 6 | 25 | >50 | 0.2 | 6 | 0.4 |
| 24 | "      " ) field | 0.02 | 6 | 1.5 | 0.1 | 0.02 | 0.2 |
| 25 | "      " ) isolates | 0.02 | 1.5 | 1.5 | 0.05 | >0.01 | 0.1 |
| 26 | "      " ) | 0.02 | 6 | 1.5 | 0.1 | >0.01 | 0.2 |

Example 5

5-week old Israeli commercial strain chickens, heavy breed, were kept for one week before the experiment on feed and water, free of additives and anti-microbial agents, ad lib.

The chickens were randomly divided into three groups of 30 each. The tested compounds were administered continuously in the drinking water at levels of 125 mg/ml of Norfloxacin base.

The medicated water was replaced every 24 hours for groups A and B and every 8 hours for group C (see below). Blood was drawn at $t_0$ (before medication), 12 hours ($t_{12}$), 24 hours ($t_{24}$) and 48 hours ($t_{48}$). Fluorescent lights were kept on throughout the experiment. Temperature monitoring and recording daily: max. 24°C, min. 15°C. The antimicrobial level in the serum for the individual chickens was determined by bioassay. HPLC determination of Norfloxacin in the serum was performed for the pooled serum in each group. Statistical analysis references: L.A. Gladstone, "Understanding Medical Statistics", William Heinemann Medical Books Ltd., London 1985.

The groups received the following compounds:

Group A      173 mg/ml Norfloxacin nicotinate 1:1 adduct

Group B      170 mg/ml Norfloxacin methane sulfonate mono hydrate

Group C      125 mg/ml Norfloxacin (by appropriate water dissolution of a composition containing 2.5 g of Norfloxacin, 45.5 ml of propylene glycol and 2 ml of monoethanol amine).

The results for group A are summarized in Table II.

Group B:

$t_0 = 0$

$t_{12}$ and $t_{24}$ In most birds no detection of serum levels. 14% of birds had serum levels at the range of 0.2 $\mu$g/ml serum.

HPLC on pooled serum 0.15 $\mu$g/ml serum ± 10%.

Group C:

Serum level of Norfloxacin at all $t_x$ were below the bioassay detection limit.

HPLC on the pooled serum was 0.05 $\mu$g/ml serum ± 10%.

Table II - Group A

$t_0$, $t_{12}$, $t_{24}$, $t_{48}$ hours post medication - serum levels
of Norfloxacin nicotinate (NORN) in $\mu$g/ml serum.
Dosage: 125 mg active ingredient/litre drinking water.

| Group | Bird number | $t_0$ | $t_{12}$ | $t_{24}$ | $t_{48}$ |
|---|---|---|---|---|---|
| | 1 | 0 | 0.12 | 0.24 | 0.20 |
| | 2 | 0 | 0.13 | 0.36 | 0.20 |
| | 3 | 0 | 0.13 | 0.24 | 0.25 |
| | 4 | 0 | 0.22 | 0.28 | 0.27 |
| | 5 | 0 | 0.23 | 0.25 | 0.27 |
| | 6 | 0 | 0.23 | 0.30 | 0.27 |
| | 7 | 0 | 0.25 | 0.26 | 0.28 |
| | 8 | 0 | 0.26 | 0.25 | 0.20 |
| | 9 | 0 | 0.26 | 0.26 | 0.40 |
| | 10 | 0 | 0.27 | 0.24 | 0.27 |
| | 11 | 0 | 0.27 | 0.28 | 0.31 |
| | 12 | 0 | 0.28 | 0.28 | 0.32 |
| | 13 | 0 | 0.29 | 0.23 | 0.32 |
| A | 14 | 0 | 0.30 | 0.23 | 0.25 |
| NORN | 15 | 0 | 0.32 | 0.47 | 0.32 |
| | 16 | 0 | 0.32 | 0.26 | 0.27 |
| | 17 | 0 | 0.33 | 0.26 | 0.42 |
| | 18 | 0 | 0.33 | 0.28 | 0.28 |
| | 19 | 0 | 0.36 | 0.28 | 0.38 |
| | 20 | 0 | 0.39 | 0.28 | 0.37 |
| | 21 | 0 | 0.40 | 0.28 | 0.20 |
| | 22 | 0 | 0.43 | 0.26 | 0.20 |
| | 23 | 0 | 0.43 | 0.23 | 0.27 |
| | 24 | 0 | 0.45 | 0.24 | 0.25 |
| | 25 | 0 | 0.48 | 0.32 | 0.32 |
| | 26 | 0 | 0.48 | 0.23 | 0.30 |
| | 27 | 0 | 0.53 | 0.24 | 0.33 |

## Table II - Group A

(Continued)

| Group | Bird number | $t_0$ | $t_{12}$ | $t_{24}$ | $t_{48}$ |
|---|---|---|---|---|---|
| | 28 | 0 | 0.58 | 0.26 | 0.27 |
| | 29 | 0 | 0.59 | 0.26 | 0.25 |
| | 30 | 0 | 0.70 | 0.23 | 0.27 |
| $\overline{X}$ | | 0 | 0.35 | 0.27 | 0.28 |
| S.D. | | 0 | 0.14 | 0.047 | 0.057 |
| S.E. | | 0 | 0.026 | 0.0086 | 0.010 |
| % response | | 0 | 100% | 100% | 100% |
| pooled serum bioassay | | 0 | N.D. | 0.28 | 0.3 |
| pooled serum HPLC | | 0 | $0.3\pm10\%$ | $0.4\pm10\%$ | $0.4\pm10\%$ |

Comments: (1) $S.E. = \dfrac{S.D.}{\sqrt{N}}$

(2) $\sqrt{N} = 5.48$

(3) S.E. = Standard error of the mean

(4) 0 = less than our detection level

(5) S.D. = Standard deviation

(6) N = Number of birds/group

(7) N.D. = not determined

Example 6

Preventive effect of the compound prepared in Example 1 against experimental systemic infection with E.coli 078 in chicken

Infection was induced by intraperitoneal injection of 0.5 McF units per kg body weight of E.coli 078 suspended in sterile 5% hog stomach mucin in 0.9% saline.

Prior to the experiment, the chickens (male, heavy breed 3 weeks old, weighing each 360-700 g) were randomly divided into groups of 30-31, and each bird was weighed individually in order to determine the exact inoculum. The chickens were acclimatized for one week before the infection process started and were kept on an antibiotic-free diet. Water was supplied ad lib.

The medicament was dissolved in the drinking water reservoirs and was replaced daily. The dosage in all groups was adjusted to a level of 173 mg (125 mg Norfloxacin base) of the compound of Example 1 per 1 litre of water.

The medicament was administered immediately post infection induction. The treatment lasted for 3 days, after which the water reservoirs were emptied and refilled with tap water.

Death was recorded daily. 6 days after the infection induction the trial was terminated. The results are summarized in Table III in which:

A stands for control infected, non treated, N = 31
B stands for the composition of Example 2, N = 31
C stands for the compound of Example 1, N = 30
D stands for the composition of Example 3, N = 30

Table III

| Date | A | B | C | D |
|---|---|---|---|---|
| 24.6.86 | Starting of the trial | | | |
| 25.6.86 | 6 | 2 | 0 | 2 |
| 26.6.86 | 3 | 0 | 0 | 1 |
| 27.6.86 | 1 | 0 | 0 | 0 |
| 28.6.86 | 3 | 0 | 0 | 0 |
| 29.6.86 | 2 | 0 | 0 | 0 |
| Total Dead | 15 | 2 | 0 | 3 |
| % Dead | 48 | 6 | 0 | 10 |

The table clearly proves that the compound prepared by Example 1 either by itself or in a formulation effectively protects chickens from E.coli infection.

At the termination of the trial, five surviving chickens from each group were euthanised and post mortem examinations were performed. Swabs from peritoneal cavity were taken and immediately applied over EMB agar. No E.coli 078 was found in the peritoneum of the treated (groups B-D) birds. In the infected non-treated control (group A) E.coli was isolated.

## Claims

**Claims for the following Contracting States : AT, BE, CH, DE, FR, GB, IT, LI, NL, SE**

1. Nicotinic acid:Norfloxacin 1:1 adduct.

2. A process for preparing an adduct as claimed in claim 1, which process comprises reacting Norfloxacin or a different adduct thereof with nicotinic acid or a salt thereof.

3. A process according to claim 2, wherein the reaction is carried out in boiling ethanol as solvent.

4. A pharmaceutical composition containing as active compound an adduct as claimed in claim 1.

5. A pharmaceutical composition according to claim 4 containing an additional active compound and/or a carrier.

6. A pharmaceutical composition according to claim 4 or claim 5 comprising a solution in water.

7. An aqueous solution containing an adduct as claimed in claim 1.

9

**8.** An adduct as claimed in claim 1 or a pharmaceutical composition as claimed in any one of claims 4 to 6, for use in a method of treatment of the human or animal body by therapy.

**9.** An adduct or a composition as claimed in claim 8, wherein the adduct is dissolved in water.

**Claims for the following Contracting State : ES**

**1.** A process for preparing a Nicotinic acid:Norfloxacin 1:1 adduct, characterized in that nicotinic acid or a salt thereof is reacted with Norfloxacin or a different adduct thereof to form nicotinic acid:Norfloxacin 1:1 adduct.

**2.** A process according to claim 1 characterized in that the reaction is carried out in the desired ratio in a suitable solvent.

**3.** A process according to claim 2 characterized in that the solvent is boiling ethanol.

**4.** A method of preparing a pharmaceutical composition characterized by bringing a nicotinic acid:Norfloxacin 1:1 adduct defined in claim 1 into a form suitable for therapeutic administration.

**Revendications**
**Revendications pour les Etats contractants suivants : AT, BE, CH, DE, FR, GB, IT, LI, NL, SE**

**1.** Produit d'addition caractérisé en ce qu'il s'agit d'un adduct acide nicotinique/norfloxacine 1/1.

**2.** Procédé pour la préparation d'un adduct tel que revendiqué dans la revendication 1, ledit procédé comprenant la réaction de la norfloxacine ou l'un de ses produits d'addition différents, avec l'acide nicotinique ou l'un de ses sels.

**3.** Procédé selon la revendication 2, dans lequel la réaction est effectuée dans l'éthanol bouillant en tant que solvant.

**4.** Composition pharmaceutique contenant en tant qu'ingrédient actif un adduct tel que revendiqué dans la revendication 1.

**5.** Composition pharmaceutique selon la revendication 4, contenant en outre un composé actif supplémentaire et/ou un véhicule.

**6.** Composition pharmaceutique selon la revendication 4 ou 5, comprenant une solution dans de l'eau.

**7.** Solution aqueuse contenant un adduct tel que revendiqué dans la revendication 1.

**8.** Utilisation en thérapeutique d'un adduct tel que revendiqué dans la revendication 1 ou d'une composition pharmaceutique telle que revendiquée dans l'une quelconque des revendications 4 à 6, destinée à l'être humain ou à l'animal.

**9.** Utilisation selon la revendication 8, dans laquelle l'adduct est dissous dans de l'eau.

**Revendications pour l'Etat contractant suivant : ES**

**1.** Procédé pour la préparation d'un adduct acide nicotinique/norfloxacine 1/1, ledit procédé étant caractérisé en ce que l'on fait réagir l'acide nicotinique ou l'un de ses sels avec la norfloxacine ou l'un de ses produits d'addition différents, de façon à former ledit adduct acide nicotinique/norfloxacine 1/1.

**2.** Procédé suivant la revendication 1, caractérisé en ce que la réaction est effectuée dans un solvant approprié selon le rapport souhaité.

**3.** Procédé selon la revendication 2, caractérisé en ce que le solvant est l'éthanol bouillant.

4. Procédé pour la préparation d'une composition pharmaceutique, caractérisé en ce que l'on amène un adduct acide nicotinique/norfloxacine 1/1, tel que défini dans la revendication 1, sous une forme appropriée pour administration thérapeutique.

**Patentansprüche**
**Patentansprüche für folgende Vertragsstaaten : AT, BE, CH, DE, FR, GB, IT, LI, NL, SE**

1. Nicotinsäure : Norfloxacin Addukt im Verhältnis 1 : 1.

2. Verfahren zur Herstellung eines Adduktes nach Anspruch 1, dadurch gekennzeichnet, dass Norfloxacin oder ein anderes Addukt davon mit Nicotinsäure oder einem ihrer Salze umgesetzt wird..

3. Verfahren nach Anspruch 2, dadurch gekennzeichnet, dass die Reaktion in siedendem Aethanol als Lösungsmittel durchgeführt wird.

4. Pharmazeutische Zusammensetzung, welche als aktive Verbindung ein Addukt nach Anspruch 1 enthält.

5. Pharmazeutische Zusammensetzung nach Anspruch 4, welche eine zusätzliche aktive Verbindung und/oder einen Träger enthält.

6. Pharmazeutische Zusammensetzung nach Anspruch 4 oder 5, welche eine Lösung in Wasser enthält.

7. Wässerige Lösung enthaltend ein Addukt nach Anspruch 1.

8. Addukt nach Anspruch 1 oder pharmazeutische Zusammensetzung nach einem der Ansprüche 4 bis 6, zur Verwendung in einem Verfahren zur therapeutischen Behandlung des menschlichen oder tierischen Körpers.

9. Addukt oder Zusammensetzung nach Anspruch 8, worin das Addukt in Wasser gelöst ist.

**Patentansprüche für folgenden Vertragsstaat : ES**

1. Verfahren zur Herstellung eines Nicotinsäure : Norfloxacin-Adduktes im Verhältnis 1 : 1, dadurch gekennzeichnet, dass Nicotinsäure oder eines ihrer Salze mit Norfloxacin oder einem anderen Addukt davon umgesetzt wird, um ein Nicotinsäure : NorfloxacinAddukt im Verhältnis 1 : 1 zu bilden.

2. Verfahren nach Anspruch 1, dadurch gekennzeichnet, dass die Reaktion im gewünschten Verhältnis in einem geeigneten Lösungsmittel durchgeführt wird.

3. Verfahren nach Anspruch 2, dadurch gekennzeichnet, dass das Lösungsmittel siedendes Aethanol ist.

4. Verfahren zur Herstellung einer pharmazeutischen Zusammensetzung, dadurch gekennzeichnet, dass ein Nicotinsäure : Norfloxacin- 1 : 1 -Addukt nach Anspruch 1 in eine zur therapeutischen Verabreichung geeignete Form gebracht wird.